(19) Europäisches Patentamt
European Patent Office
Office européen des brevets

(11) EP 1 473 018 B1

(12) # FASCICULE DE BREVET EUROPEEN

(45) Date de publication et mention de la délivrance du brevet:
**20.01.2010 Bulletin 2010/03**

(51) Int Cl.:
***A61Q 1/12*** *(2006.01)*

(21) Numéro de dépôt: **04290917.6**

(22) Date de dépôt: **06.04.2004**

(54) **Composition cosmétique comprenant une poudre absorbant le sébum et une poudre à faible énergie de surface critique**

Kosmetische Zubereitung enthaltend ein Pulver zum Absorbieren von Sebum sowie ein eine niedrige Oberflächenenergie aufweisendes Pulver

Cosmetic composition comprising a powder capable of absorbing sebum and a powder with a low critical surface energy

(84) Etats contractants désignés:
**AT BE BG CH CY CZ DE DK EE ES FI FR GB GR HU IE IT LI LU MC NL PL PT RO SE SI SK TR**

(30) Priorité: **28.04.2003 FR 0305174**

(43) Date de publication de la demande:
**03.11.2004 Bulletin 2004/45**

(73) Titulaire: **L'ORÉAL**
**75008 Paris (FR)**

(72) Inventeurs:
- **Auguste, Frédéric**
  **94550 Chevilly-Larue (FR)**
- **Hadasch, Anke**
  **75013 Paris (FR)**

(74) Mandataire: **Kromer, Christophe**
**L'Oréal**
**D.I.P.I.**
**25-29 Quai Aulagnier**
**92600 Asnières (FR)**

(56) Documents cités:
**EP-A- 0 847 752** **EP-A- 1 116 753**
**EP-A- 1 206 928** **WO-A-96/17583**
**WO-A-97/04737** **FR-A- 2 675 377**

- **DATABASE WPI Section Ch, Week 200334 Derwent Publications Ltd., London, GB; Class A25, AN 2003-358371 XP002266458 & JP 2003 055134 A (SHISEIDO CO LTD) 26 février 2003 (2003-02-26)**
- **DATABASE WPI Section Ch, Week 199325 Derwent Publications Ltd., London, GB; Class D21, AN 1993-200415 XP002266459 & JP 05 124932 A (KAO CORP) 21 mai 1993 (1993-05-21)**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour objet une composition cosmétique comprenant un mélange de poudres. L'invention a également pour objet un procédé de maquillage ou de soin des matières kératiniques comme la peau, les lèvres, les ongles, les cheveux d'être humain comprenant l'application de la composition sur les matières kératiniques.

La composition selon l'invention peut être une composition de maquillage et/ou de soin des matières kératiniques, en particulier une composition de maquillage de la peau, telle qu'un fond de teint, un fard à paupières, un fard à joue, un produit anti-cernes, une poudre du visage et du corps, un produit de maquillage du corps.

Les poudres de maquillage comprennent généralement, d'une part, une phase pulvérulente comportant notamment des pigments et des charges et d'autre part, une phase grasse au titre de liant comprenant des corps gras, destinée à conférer au produit fini une certaine densité, à donner une douceur et une propriété émolliente au produit de maquillage et à favoriser son adhérence sur la peau.

Après l'application d'un maquillage sur la peau, le sébum excrété par la peau au cours du temps modifie les propriétés cosmétiques du maquillage. En particulier, le sébum ne favorise pas l'adhésion du maquillage sur la peau : le maquillage a tendance à transférer sur les surfaces ou les tissus mis en en contact avec la peau maquillé engendrant une perte du maquillage restant sur la peau. En outre, le maquillage imprégné de sébum a tendance à migrer plus facilement dans les plis ou les rides du visage rendant le dépôt de maquillage non homogène. De plus, le sébum rend le maquillage plus sensible aux frottements, notamment aux frottements des tissus, des doigts provoquant une élimination du maquillage au cours du temps. La mauvaise tenue du maquillage au cours de la journée nécessite à l'utilisatrice de renouveler l'application du maquillage pour conserver un maquillage homogène.

Par ailleurs, le sébum a tendance à modifier la couleur du maquillage déposé sur la peau, la couleur devient plus foncée; cette modification de la couleur va à l'encontre de l'effet esthétique coloré recherché puisque l'aspect visuel du maquillage ne correspond plus à la couleur initiale de la composition choisie. Le maquillage devient également plus brillant ; or cet aspect brillant n'est pas recherché pour un fond de teint, les consommatrices préférant un maquillage mat et conservant cette matité pendant la journée.

Il est connu du document WO 97/04737 d'employer des poudres absorbant le sébum en mélange avec des charges et/ou des pigments dans des compositions de poudres de maquillage. Il est aussi connu du document WO 9617583 d'utiliser de la silice sphérique adsorbée par un agent hydratant pour supprimer la brillance de la peau tout en maintenant une effet hydratant. Le document JP 200055134 décrit une composition cosmétique contenant une poudre composite formée de particules de gomme de silicone enrobées de résine polyorganosylsesquioxane, une silice modifiée polyéther, de la poudre de silice et d'une huile de silicone. Le document EP 1116753 décrit une poudre traitée en surface par une composé siliconé, utilisée dans une composition cosmétique. Le document WO 9704737 décrit une poudre cosmétique comprenant du talc, du dioxyde de titane et une poudre absorbant le sébum. Toutefois, les charges et les pigments peuvent également s'imprégner de sébum et l'adsorption du sébum ne se fait pas de manière sélective par les poudres absorbant le sébum. Les inconvénients dus à l'excrétion du sébum au cours du temps subsistent donc pour ces compositions, notamment la modification de la couleur et l'aspect brillant du maquillage.

Le but de la présente invention est donc de disposer d'une composition de maquillage pouvant absorber efficacement le sébum et donc de conférer un maquillage présentant de bonne propriétés cosmétiques au cours du temps, en particulier présentant une bonne tenue aux frottements, des propriétés non transfert, une bonne homogénéité, un maintien de la couleur initiale du maquillage, une absence de brillance (tenue de la matité).

Les inventeurs ont découvert qu'une telle composition est obtenue en employant une poudre absorbant le sébum associée avec une poudre ayant une énergie de surface critique particulière. Cette poudre à énergie de surface critique particulière ne s'imprègne pas de sébum lorsque le maquillage est déposé sur la peau et empêche donc au sébum excrété au cours du temps de modifier les propriétés cosmétiques du maquillage.

De façon plus précise, l'invention a pour objet une composition cosmétique comprenant une phase pulvérulente contenant au moins une première poudre absorbant le sébum ayant une prise de sébum, une deuxième poudre ayant une énergie de surface critique inférieure ou égale à 24 mN/m, et une phase grasse liquide liante comprenant une première huile non volatile ayant des paramètres de solubilité de Hansen $\delta^L{}_d$, $\delta^L{}_p$, $\delta^L{}_h$, exprimés en $J^{1/2}.\ cm^{-3/2}$, tels que

$$\Delta\delta = \sqrt{4\left(16.4 - \delta_d^L\right)^2 + \left(0.9 - \delta_p^L\right)^2 + \left(4.2 - \delta_h^L\right)^2}$$

et $\Delta\delta$ va de 6 à 20.

L'invention a également pour objet un procédé cosmétique de maquillage ou de soin non thérapeutique des matières kératiniques comprenant l'application sur les matières kératiniques d'une composition telle que définie précédemment. Les matières kératiniques sont en particulier la peau d'être humain.

L'invention a aussi pour objet l'utilisation d'une composition telle que définie précédemment pour obtenir un

maquillage de la peau non brillant et/ou homogène et/ou non transfert et/ou résistant aux frottements et/ou présentant un maintien de la couleur initiale.

L'invention a encore pour objet l'utilisation d'une première poudre absorbant le sébum ayant une prise de sébum, et d'une deuxième poudre ayant une énergie de surface critique inférieure ou égale à 24 mN/m, dans une composition cosmétique, pour obtenir un maquillage de la peau non brillant et/ou homogène et/ou non transfert et/ou résistant aux frottements et/ou présentant un maintien de la couleur initiale.

La composition selon l'invention contient une poudre absorbant le sébum ayant une prise de sébum. Avantageusement, la prise de sébum de la poudre est supérieure ou égale à 1 ml/g, notamment allant de 1 ml/g à 20 ml/g, voire allant de 1 ml/g à 15 ml/g. De préférence, la poudre à une prise de sébum supérieure ou égale à 1,5 ml/g, notamment allant de 1,5 ml/g à 20 ml/g, voire allant de 1,5 ml/g à 15 ml/g, et préférentiellement supérieure ou égale à 2 ml/g, notamment allant de 2 ml/g à 20 ml/g, voire allant de 2 ml/g à 15 ml/g.

On entend par poudre absorbant le sébum une poudre apte à absorber et/ou adsorber le sébum.
La prise de sébum correspond à la quantité de sébum absorbé et/ou adsorbé par la poudre. Elle est mesuré selon la méthode du Wet Point décrit ci-après.

Avantageusement, la poudre absorbant le sébum peut avoir une surface spécifique BET supérieure ou égale à 300 $m^2/g$, de préférence supérieure à 500 $m^2/g$, et préférentiellement supérieure à 600 $m^2/g$, et notamment inférieure à 1500 $m^2/g$.

La « surface spécifique BET » est déterminée selon la méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale (donc micropores compris) de la poudre.

La poudre absorbant le sébum peut être une poudre minérale ou une poudre organique ; elle peut être choisie parmi la silice, les poudres de polyamides (nylon®), les poudres de polymères acryliques, notamment de polyméthacrylate de méthyle, de poly méthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle ; les poudres de silicone élastomère, notamment obtenues par polymérisation d'organopolysiloxane ayant au moins deux atomes d'hydrogène liés chacun à un atome de silicium et d'un organopolysiloxane comprenant au moins deux groupes à insaturation éthylénique (notamment deux groupes vinyles) en présence de catalyseur platine.

La poudre absorbant le sébum peut être une poudre enrobée avec un agent de traitement hydrophobe.

L'agent de traitement hydrophobe peut être choisi parmi les acides gras comme l'acide stéarique ; les savons métalliques comme le dimyristate d'aluminium, le sel d'aluminium du glutamate de suif hydrogéné ; les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium.
L'acide aminé peut être par exemple la lysine, l'acide glutamique, l'alanine.

Le terme alkyl mentionné dans les composés cités précédemment désigne notamment un groupe alkyle ayant de 1 à 30 atomes de carbone, de préférence ayant de 5 à 16 atomes de carbone.

Comme poudre de silice, on peut citer :

- les microsphères de silice poreuses vendues sous la dénomination SILICA BEADS SB-700 par la société MYOSHI ; "SUNSPHERE® H51", "SUNSPHERE® H33" par la société ASAHI GLASS ;
- les microsphères de silice amorphe enrobées de polydiméthylsiloxane vendues sous la dénomination "SA SUNSPHERE® H 33", "SA SUNSPHERE® H53" par la société ASAHI GLASS.

Comme poudre de polymères acryliques, on peut citer :

- les poudres de polyméthacrylate de méthyle vendus sous la dénomination COVABEAD® LH85 par la société WACKHERR ;
- les poudres de poly méthacrylate de méthyle/diméthacrylate d'éthylène glycol vendues sous la dénomination DOW CORNING 5640 MICROSPONGE® SKIN OIL ADSORBER par la société DOW CORNING ; GANZPEARL® GMP-0820 par la société GANZ CHEMICAL ;
- les poudres de polyméthacrylate d'allyle/diméthacrylate d'éthylène glycol vendues sous la dénomination POLY-PORE® L200, POLY-PORE® E200 par la société AMCOL ;
- les poudres de copolymère diméthacrylate d'éthylène glycol/méthacrylate de lauryle vendues sous la dénomination POLYTRAP® 6603 de la société DOW CORNING.

Comme poudre de nylon, on peut citer la poudre de nylon vendue sous la dénomination ORGASOL® 4000

par la société ATOCHEM.

Comme poudre de silicone élastomère, on peut citer les poudres vendues sous les dénominations "Trefil® Powder E-505C", "Trefil® Powder E-506C" par la société DOW CORNING.

La poudre absorbant le sébum particulièrement préférée est la poudre de silice, notamment ayant les caractéristiques décrites précédemment, en particulier ayant une prise de sébum supérieure ou égale à 2 ml/g, notamment allant de 2 ml/g 20 à ml/g. Une telle poudre de silice est notamment vendue sous la dénomination "SUNSPHERE® H 33" par la société ASAHI GLASS.

La poudre absorbant le sébum peut être présente dans la composition selon l'invention en une teneur allant de 1 % à 98 % en poids, par rapport au poids total de la composition, de préférence allant de 5 % à 80 % en poids, et préférentiellement allant de 5 % à 60 % en poids ou bien encore allant de 1 % à 15 % en poids.

La composition selon l'invention contient également une poudre, dite deuxième poudre, ayant une énergie de surface critique inférieure ou égale à 24 mN/m (notamment allant de 10 à 24 mN/m), et de préférence inférieure ou égale à 20 mN/m (notamment allant de 10 à 20 mN/m).

L'énergie de surface critique d'une poudre est mesurée selon la méthode décrite ci-après.

La deuxième poudre peut être une poudre enrobée d'un composé organique fluoré ou d'un composé siliconé.

La poudre (destinée à être enrobée) peut être choisie parmi les pigments, les nacres et les charges habituellement utilisés dans les compositions cosmétiques, telles que celles décrites ci-après dans la partie descriptive des poudres additionnelles.

Le composé organique fluoré enrobant la poudre peut être un perfluoroalkylphosphate ou un polyoxyde d'hexafluoropropylène.

Des poudres enrobées de perfluoroalkyle phosphate sont notamment décrites dans les documents US 3632744, JP-A- 04-330007, JP-A-2001-316223.

Le perfluoroalkyle phosphate peut notamment être choisi parmi ceux de formule (I)

$$[C_mF_{2m+1}C_nH_{2n}O]_yPO(OM)_{3-y}$$

avec m entier allant de 1 à 21 ; n entier allant de 1 à 14 ; y = 1, 2 ou 3 ; M est -H, un cation de métal alcalin, un groupe ammonium ou un groupe ammonium substitué par un à trois radicaux alkyle en $C_1$-$C_8$.

Selon un autre mode de réalisation, la deuxième poudre peut être une poudre enrobée d'un composé siliconé, comme les méthicones, les diméthicones, les polyorganosiloxanes comprenant des groupes perfluoroalkyles perfluoropolyéthers, les perfluoroalkyl silanes.

Comme poudre enrobée ayant une énergie de surface critique telle que définie précédemment, on peut utiliser :

- les poudres enrobées de phosphate de perfluoroalkyle et de copolymère acrylate de butyle/acrylate de perfluoroalkyl ($C_6$-$C_{14}$) éthyle/mercaptopropyldiméthicone vendues sous les dénominations "NOVATECH NFP Double Treated Talc ", "NOVATECH NFP Double Treated Sericite" , "NOVATECH NFP Double Treated Yellow Iron Oxide", "NOVATECH NFP Double Treated Red Iron Oxide", "NOVATECH NFP Double Treated Black Iron Oxide", "NOVATECH NFP Double Treated Titanium Dioxide" par la société DAIKIN ;

- les poudres enrobées de phosphate de perfluoroalkyle, de copolymère acrylate de butyle/acrylate de perfluoroalkyl ($C_6$-$C_{14}$) éthyle/mercaptopropyldiméthicone et de copolymère méthacrylate d'hydroxyéthyl/acrylate de perfluoro alkyl($C_6$-$C_{14}$) éthyle vendues sous les dénominations "NOVATECH NFP Triple Treated Talc ", "NOVATECH NFP Triple Treated Sericite" , "NOVATECH NFP Triple Treated Yellow Iron Oxide ", "NOVATECH NFP Triple Treated Red Iron Oxide ", "NOVATECH NFP Triple Treated Black Iron Oxide", "NOVATECH NFP Triple Treated Titanium Dioxide " par la société DAIKIN .

Le composé organique fluoré ou le composé siliconé enrobant la poudre peut être présent en une teneur allant de 0,01 % à 60 % en poids, par rapport au poids total de la poudre enrobée, de préférence allant de 0,05 % à 40 % en poids, et préférentiellement allant de 0,1 % à 40 % en poids.

La deuxième poudre telle que décrite précédemment peut être présente dans la composition selon l'invention en une teneur allant de 0,1 % à 95 % en poids, par rapport au poids total de la composition, de préférence allant de 1 % à 90 % en poids, et préférentiellement allant de 5 % à 80 % en poids.

La phase liante de la composition favorise la bonne dispersion des poudres. Cette phase liante comprend une phase grasse liquide à température ambiante (25 °C), comprenant une première huile non volatile telle que décrite précédemment.

La phase grasse liquide peut être présente en une teneur allant de 0,1 % à 90 % en poids, par rapport au poids total de la composition, de préférence allant de 0,1 % à 75 % en poids, et préférentiellement allant de 1 % à 50 % en poids, et mieux allant de 1 % à 30 % en poids.

La phase grasse liquide comprend une ou plusieurs huiles non volatiles.

On entend par huile non volatile une huile susceptible de rester sur la peau à température ambiante (25 °C) et pression atmosphérique au moins une heure et ayant notamment une pression de vapeur à température ambiante (25 °C) et pression atmosphérique, non nulle, inférieure ou égale à 0,01 mm de Hg (1,33 Pa).

Avantageusement, la phase grasse liquide non volatile peut comprendre une première huile non volatile ayant une viscosité, mesurée à 25 °C, supérieure ou égale à 5 x $10^{-2}$ Pa.s (50 cps), notamment allant de 5 x $10^{-2}$ Pà.s à 40 Pa.s (40 000 cps ), et de préférence supérieure ou égale à 9 x $10^{-2}$ Pa.s (90 cps), notamment allant de 9 x $10^{-2}$ Pa.s à 40 Pa.s.

La viscosité est mesurée à 25 °C $\pm$ 0,5 °C à l'aide d'un rhéomètre à contrainte imposée Haake RS75 de la société Thermo Rhéo équipé d'un mobile de géométrie cône/plan d'un diamètre compris entre 2 cm et 6 cm et d'un angle compris entre 1 ° et 2°, le choix du mobile étant fonction de la viscosité à mesurer (plus l'huile est fluide, plus le diamètre du cône choisi est grand et plus l'angle est petit). La mesure est effectuée en imposant à l'échantillon d'huile une rampe logarithmique de contrainte de cisaillement $\tau$ allant de 0,2 Pa à 1000 Pa pendant une durée de 20 minutes. Puis on trace le rhéogramme représentant l'évolution de la viscosité en fonction de la vitesse de cisaillement $\varepsilon'$ . Le rhéogramme présente un plateau aux valeurs basses de vitesse de cisaillement (dit plateau newtonien) ; ce plateau correspond à une valeur stable de viscosité qui est la viscosité de l'huile ainsi déterminée.

La phase grasse liquide non volatile comprend une première huile non volatile ou un mélange de premières huiles non volatiles.

La phase grasse liquide liante comprend au moins une première huile non volatile ayant des paramètres de solubilité de Hansen $\delta^L{}_d$, $\delta^L{}_p$, $\delta^L{}_h$ , exprimés en $J^{1/2}$. $cm^{-3/2}$ , tels que

$$\Delta\delta = \sqrt{4\left(16.4-\delta_d^L\right)^2+\left(0.9-\delta_p^L\right)^2+\left(4.2-\delta_h^L\right)^2}$$

et $\Delta\delta$ va de 6 à 20.

De préférence, la première huile non volatile a des paramètres de solubilité de Hansen tels que $\Delta\delta$ va de 6 à 16.

Avantageusement, $\delta^L{}_d$ est inférieur ou égal à 17, de préférence inférieur ou égal à 14.

Les paramètres de solubilité sont les paramètres de solubilité de Hansen tels que définis dans l'article de C. M. HANSEN "The three dimensional solubility parameters"", J. paint Technol. 39, 105 (1967) et dans l'ouvrage suivant : "Handbook of solubility Parameters", Allan F. BARTON, CRC Press, 1991.

- $\delta^L{}_p$ est la composante polaire du paramètre de solubilité de la première huile non volatile,
- $\delta^L{}_h$ est la composante des liaisons spécifiques (liaisons hydrogène) du paramètre de solubilité de la première huile non volatile.

La première huile non volatile telle que décrite précédemment peut être notamment choisie parmi le citrate de tri-iso arachidyle , le poly vinylpyrrolidone / hexadécène de poids moléculaire moyen en poids allant de 5000 à 9000 (tel que celui vendu sous la dénomination ANTARON® V-216 par la société ISP), le trimellitate de butyl-2 octanol , le citrate de triisostéaryle, le trimellitate de tridécyle, le polyglycéryl-2 triisostéarate, le tétra-éthyl-2 hexanoate de pentaérythrityle, le trimellitate de triisodécyle, le malate de diisostéaryle, le trimellitate de tri-éthyl-2-hexyle, le 2-octyl-dodécanol, l'octyl hydroxystéarate, le polybutylène de poids moléculaire moyen en poids allant de 800 à 1200 (tel que celui vendu sous la dénomination INDOPOL® H-100 par la société AMOCO), le lactate d'isostéaryle, le monoisostéarate de propylène glycol, le polyglycéryl-2 diisostéarate, l'huile de ricin, le dibenzoate de dipropylène glycol, le triacétate de glycéryle oxyéthyléné (7 OE), le polyglycéryl 3 diisostéarate, le poly méthyltrifluoropropyl diméthylsiloxane de formule (I) :

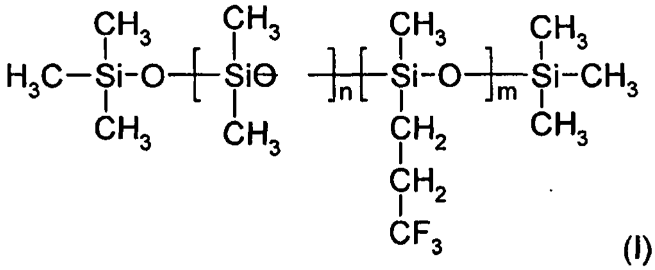

(I)

dans laquelle n est un entier allant de 5 à 90, de préférence allant de 30 à 80 et mieux allant de 50 à 80, et m est un entier allant de 1 à 150, de préférence allant de 1 à 80, et mieux allant de 1 à 40,
a est un entier allant de 0 à 5, Rf désigne un radical perfluoroalkyle ayant de 1 à 8 atomes de carbone.
Comme composé de formule (I), on peut citer ceux vendus sous la dénomination X22-819, X22-820, X22-821, X22-822 par la société SHIN-ETSU.

La première huile non volatile particulièrement préférée est le poly méthyltrifluoropropyl diméthylsiloxane de formule (I) défini précédemment. En particulier, cette première huile non volatile préférée peut être utilisée avec la poudre de silice absorbant le sébum préférée décrite précédemment.

La première huile non volatile telle que définie précédemment peut être présente dans la composition selon l'invention en une teneur allant de 0,1 % à 90 % en poids, par rapport au poids total de la composition, de préférence allant de 0,1 % à 75 % en poids, et préférentiellement allant de 1 % à 50 % en poids, et mieux allant de 1 % à 30 % en poids.

Selon un mode de réalisation particulier de la composition selon l'invention, la phase grasse liquide peut comprendre 100 % en poids de la première huile non volatile telle que définie précédemment ou d'un mélange de telles premières huiles.

La phase grasse liquide de la composition selon l'invention peut comprendre une deuxième huile non volatile différente de la première huile non volatile décrite précédemment. En particulier, la deuxième huile non volatile a des paramètres de Hansen tels que $\Delta\delta$ soit inférieur à 2 ou supérieur à 16 et/ou une viscosité inférieure à 5 x $10^{-2}$ Pa.s.

La deuxième huile non volatile peut être une huile hydrocarbonée, une huile siliconée, une huile fluorée.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Comme deuxième huile non volatile, on peut citer l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de jojoba, l'huile d'arachide ; les huiles d'hydrocarbures, telles que les huiles de paraffine, le squalane, la vaseline ; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyldécyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine ; les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tel que l'alcool oléique ; et leurs mélanges.

Lorsqu'elle la composition selon l'invention comprend une deuxième huile non volatile, cette deuxième huile non volatile est présente en une quantité en volume inférieure à 0,5 x (prise de sébum de poudre absorbant le sébum) x (quantité en poids de poudre absorbant le sébum présente dans la composition), de préférence inférieure à 0,4 x (prise de sébum de poudre absorbant le sébum) x (quantité en poids de poudre absorbant le sébum présente dans la composition), et préférentiellement inférieure à 0,3 x (prise de sébum de poudre absorbant le sébum) x (quantité en poids de poudre absorbant le sébum présente dans la composition).

Comme huile non volatile, on peut également utiliser des huiles perfluoropolyéthers telles que le perfluoro poly méthylisopropyl éther de poids moléculaire moyen en poids de 1500 vendu sous la dénomination "FOMBLIN® HC04" par la société AUSIMONT.

La phase grasse non volatile, notamment constituée de la première huile non volatile ou d'un mélange de premières huiles non volatiles, et éventuellement de la deuxième huile volatile, ou d'un mélange de deuxièmes huiles non volatiles, peut être présente dans la composition selon l'invention en une teneur allant de 0,1 % à 90 % en poids, par rapport au poids total de la composition, de préférence allant de 0,1 % à 75 % en poids, et préférentiellement allant de 1 % à 50 % en poids, et mieux allant de 1 % à 30 % en poids.

La phase grasse liquide peut comprendre au moins une huile volatile.

Par huile volatile , on entend une huile susceptible de s'évaporer de la peau, en moins d'une heure à température ambiante et pression atmosphérique. Cette huile a notamment une pression de vapeur, à température ambiante (25°C) et pression atmosphérique (760 mm Hg) supérieure à 0,01 et inférieure ou égale à 300 mm de Hg (1,33 Pa à 40 000 Pa) et de préférence allant de 0,05 à 300 mm de Hg (6,65 Pa à 40 000 Pa).

L'huile volatile peut être choisie parmi les huiles d'origine minérale, animale, végétale ou synthétique, carbonées, hydrocarbonées, fluorées et/ou siliconées, seules ou en mélange.

Comme huile volatile, on peut citer les huiles de silicones linéaires ou cycliques ayant une viscosité à température ambiante inférieure à 8 $mm^2/s$ et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyl trisiloxane, l'heptaméthyl octyl trisiloxane, l'hexaméthyl disiloxane, l'octaméthyl trisiloxane, le décaméthyl tétrasiloxane, le dodécaméthyl pentasiloxane et leurs mélanges.

Comme autre huile volatile utilisable dans l'invention, on peut citer les huiles volatiles hydrocarbonées ayant

de 8 à 16 atomes de carbone et leurs mélanges et notamment les alcanes ramifiés en $C_8$-$C_{16}$ comme les isoalcanes (appelées aussi isoparaffines) en $C_8$-$C_{16}$, l'isododécane, l'isodécane, l'isohexadécane et par exemple les huiles vendues sous les noms commerciaux d'Isopars' ou de Permethyls', les esters ramifiés en $C_8$-$C_{16}$ comme le néo pentanoate d'iso-hexyle et leurs mélanges. On utilise de préférence l'isododécane.

L'huile volatile peut être présente en une teneur allant de 0,1 % à 90 % en poids, par rapport au poids total de la composition, de préférence allant de 0,1 % à 75 % en poids, et préférentiellement allant de 1 % à 50 % en poids.

La composition selon l'invention peut comprendre une phase aqueuse contenant de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants autres que l'eau comme par exemple les alcools primaires tels que l'éthanol et l'isopropanol, les glycols tels que le glycérol, le propylène glycol, le butylène glycol, le dipropylène glycol, le diéthylène glycol, les éthers de glycol tel que les alkyl($C_1$-$C_4$)éther de mono, di- ou tripropylène glycol, mono, di- ou triéthylène glycol, et leurs mélanges.

La phase aqueuse peut comprendre en outre des agents de stabilisation , par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensio-actifs et leurs mélanges.

De préférence, la phase aqueuse, et notamment l'eau, peut être présente dans la composition selon l'invention en une teneur allant de 0,1 % à 60 %, en poids, de préférence allant de 5 % à 50 % en poids, par rapport au poids total de la composition.

Selon un mode particulier de réalisation de l'invention, la phase liante peut comprendre une phase aqueuse telle que décrite précédemment.

Selon un autre mode de réalisation de l'invention, la composition peut être une composition anhydre, c'est-à-dire une composition contenant moins de 2 % en poids d'eau, voire moins de 0,5 % d'eau, l'eau n'étant pas ajoutée lors de la préparation de la composition mais correspondant à l'eau résiduelle apportée par les ingrédients mélangés.

La phase pulvérulente de la composition selon l'invention peut contenir des poudres additionnelles différentes des poudres absorbant le sébum et des poudres ayant une énergie de surface critique inférieure ou égale à 24 mN/m décrites précédemment. Ces poudres additionnelles ont donc une prise de sébum inférieure à 1 ml/g et une énergie de surface critique supérieure à 24 mN/m.

La poudre additionnelle peut être choisie parmi les pigments, les nacres, les charges.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être présents dans la composition, en un teneur allant de 0,01 % à 30 % en poids, par rapport au poids de la composition, de préférence allant de 1 % à 20 % en poids.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les nacres peuvent être présentes dans la composition en une teneur allant de 0,01 % à 50 % en poids, par rapport au poids total de la composition, de préférence allant de 0,1 % à 30 % en poids.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc) . On peut citer le talc, le mica, la silice, le kaolin, les poudres de polyamide (Nylon®) , de poly-β-alanine et de polyéthylène, la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant

de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent être présentes dans la composition en une teneur allant de 0,01 % à 98,9 % en poids, par rapport au poids total de la composition, de préférence allant de 0,1 % à 85 % en poids.

La poudre additionnelle peut être présente dans la composition en une teneur allant de 0,01 à 98,9 % en poids, par rapport au poids total de la composition, de préférence allant de 0,1 % à 85 % en poids, et préférentiellement allant de 1 % à 70 % en poids.

La composition peut contenir d'autres ingrédients cosmétiques usuels pouvant être choisis notamment parmi les antioxydants, les parfums, les conservateurs, les neutralisants, les tensioactifs, les cires, les filtres solaires, les vitamines, les hydratants, les composés auto-bronzants, les actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'une poudre compacte, d'une poudre pressée, d'une poudre coulée, d'une poudre libre. Elle peut également se présenter sous forme d'un gel, d'une émulsion eau-dans-huile, huile-dans-eau, d'une émulsion multiple, d'un lait, d'une pâte.

L'invention est illustrée plus en détails par les exemples décrits ci-après.

**<u>Méthode de mesure de prise de sébum d'une poudre</u>** :

La prise de sébum d'une poudre est mesurée selon la méthode de détermination de prise d'huile de poudre décrite dans la norme NF T 30-022. Elle correspond à la quantité de sébum adsorbé sur la surface disponible de la poudre par mesure du Wet Point.

On place une quantité m (en grammes) de poudre comprise entre environ 0,5 g et 5 g (la quantité dépend de la densité de la poudre) sur une plaque de verre puis on ajoute goutte à goutte du sébum artificiel ayant la composition suivante :

- trioléine 29 %
- acide oléïque 28,5 %
- oléate d'oléyle 18,5 %
- squalène 14 %
- cholestérol 7 %
- palmitate de cholestérol 3 %

Après addition de 4 à 5 gouttes de sébum artificiel, on incorpore le sébum artificiel dans la poudre à l'aide d'une spatule et on continue d'ajouter du sébum artificiel jusqu'à la formation de conglomérats de sébum artificiel et de poudre. A partir de ce moment, on ajoute le sébum artificiel à raison d'un goutte à la fois et on triture ensuite le mélange avec la spatule. On cesse l'addition de sébum artificiel lorsque l'on obtient une pâte ferme et lisse. Cette pâte doit se laisser étendre sur la plaque de verre sans craquelures ni formation de grumeaux. On note alors le volume Vs (exprimé en ml) de sébum artificiel utilisé.

La prise de sébum correspond au rapport Vs / m.

**<u>Méthode de mesure de l'énergie de surface critique critique d'une poudre</u> :**

La poudre est compactée avec une pastilleuse , de type Manual Hydraulic Press, fabriquée par Perkin-Elmer (utilisée pour réaliser les échantillons analysés en spectroscopie infra-rouge). La pression de compactage est de 10 tonnes. La pastille de poudre compactée a un diamètre de l'ordre de 10 mm et une épaisseur d'environ 1 mm.
Les pastilles de poudre compactée sont posées sur le porte-échantillon d'un tensiomètre à angle de contact tel que le tensiomètre DAT 1100, commercialisé par la Société Fibro (Suède).
Les mesures d'angles de contact sont réalisées à 27 +/- 1 °C avec les 3 solvants suivants : eau, diiodométhane et formamide.
Pour chacun des solvants, une goutte de solvant, de volume compris entre 2 et 10 microlitres, est déposée sur la pastille de poudre. Le système optique et d'analyse d'images du tensiomètre permettent de suivre l'évolution au cours du temps de l'étalement de la goutte. L'angle de contact du liquide sur la poudre compactée correspond à l'angle compris entre la surface de contact poudre/liquide et la tangente à la goutte passant par le point de rencontre de la goutte, de l'air et de la surface.

La valeur de l'angle de contact utilisée pour la détermination de l'énergie de surface critique critique est la moyenne des angles de contact mesurés sur 5 à 10 pastilles de poudre, les valeurs de l'angle étant prises après un

temps de contact goutte de solvant / pastille de poudre égal à 1 seconde.
Pour la détermination de l'énergie de surface critique critique, on représente sur un graphe l'évolution du cosinus de l'angle de contact en fonction de la tension de surface du solvant associé, pour les 3 solvants testés.
L'énergie de surface critique critique de la poudre correspond alors à la tension de surface telle que le cosinus de l'angle de contact est égal à 1 lorsqu'on extrapole de façon linéaire l'évolution de l'angle de contact en fonction de la tension de surface.

**Exemple 1 :**

On a préparé une poudre de maquillage ayant la composition suivante :

| | |
|---|---|
| Talc enrobé de phosphate de perfluoroalkyle ("NOVATECH NFP TALC-N2" de DAIKIN) | 35,4 g |
| Séricite enrobée ("NOVATECH NFP Triple treated sericite" de DAI KI N) | 30 g |
| Silice poreuse (Sunsphère® H33 de ASAHI GLASS) | 10 g |
| Oxyde de fer jaune | 5 g |
| Oxyde de fer brun | 3 g |
| Bleu d'Outremer | 3,7 g |
| Dioxyde de titane | 2,9 g |
| Silicone fluorée* (X22-819 de Shin-Etsu) | 10 g |

* poly méthyltrifluoropropyl diméthylsiloxane

Après application de cette poudre sur la peau, on obtient un maquillage présentant une bonne aptitude à absorber le sébum excrété par la peau au cours de la journée, restant bien homogène, non brillant et conservant sa couleur initiale.

**Exemple 2 :**

On a préparé un fond de teint (émulsion E/H) ayant la composition suivante :

- Silicone fluorée* (X22-819 de Shin-Etsu) 3,9 g
- Cyclopentasiloxane 15,5 g
- Polydimethylsiloxane 2,0 g
- Bentonite 2,5 g
- mélange de cétyl diméthicone copolyol, d'isostéarate de polyglycérol-4 et de laurate d'hexyle 40/30/30 (ABIL® WE 09 de Goldschmidt) 1,7 g
- Oxyde de fer jaune enrobé ("NOVATECH NFP Double Treated Yellow Iron Oxide" de DAIKIN) 1,0 g
- Oxyde de fer brun enrobé ("NOVATECH NFP Double Treated Red Iron Oxide" de DAIKIN) 0,4 g
- Oxyde de fer noir enrobé ("NOVATECH NFP Double Treated Black Iron Oxide" de DAIKIN) 0,1 g
- Dioxyde de titane enrobé ("NOVATECH NFP Double Treated Titanium Dioxide" de DAIKIN) 3,9 g
- Silice poreuse (Sunsphere® H51 de ASAHI GLASS) 4,0 g
- Séricite enrobée ("NOVATECH NFP Triple treated sericite" de DAIKIN) 6,0 g

- Eau 48,0 g
- Glycérine 5,0 g
- Ethanol 5,0 g
- Conservateur 1,0 g

* poly méthyltrifluoropropyl diméthylsiloxane

Après application de ce fond de teint sur la peau, on obtient un maquillage présentant une bonne aptitude à absorber le sébum excrété par la peau au cours de la journée, restant bien homogène, non brillant et conservant la couleur.

**Exemple 3 :**

On a préparé un fond de teint (émulsion H/E) ayant la composition suivante :

- Polybutylène (INDOPOL H100 de AMOCO) 1,0 g

- Silicone fluorée* (X22-819 de Shin-Etsu) 2,0 g
- Isododécane 19,0 g

- Lauroyl sarcosinate de sodium 1,0 g
- Acide stéarique 2,0 g
- stéarate de glycéryle 2,6 g
- isostéarate de glycéryle 2,0 g
- Triéthanolamine 1,0 g

- Oxyde de fer jaune 1,0 g
- Oxyde de fer brun 0,4 g
- Oxyde de fer noir 0,1 g
- Dioxyde de titane 3,9 g
- Silice poreuse (Sunsphere® H53 de ASAHI GLASS) 5,0 g
- Talc enrobé ("NOVATECH NFP Triple treated TALC" de DAIKIN) 5,0 g

- Eau 53,0 g
- Conservateur 1,0 g

Après application de ce fond de teint sur la peau, on obtient un maquillage présentant une bonne aptitude à absorber le sébum excrété par la peau au cours de la journée, restant bien homogène, non brillant et conservant la couleur.

**Exemple 4 :**

On prépare un fond de teint coulé anhydre ayant la composition suivante :

- Cire de polyéthylène (Polywax 500 de Bareco) 4,0 g
- Cire de polyéthylène (Performalene 400 Polyéthylene de New Phase Technologies) 8,0 g
- Cyclohexadiméthylsiloxane 20,0 g
- Cyclopentadiméthylsiloxane 38,0 g
- Silicone fluorée* (X22-819 de Shin-Etsu) 5,0 g

- Oxyde de fer jaune 2,2 g
- Oxyde de fer brun 0,6 g
- Bleu d'Outremer 0,3 g
- Dioxyde de titane 7,0 g

- Silice poreuse (Sunsphère® H53 de ASAHI GLASS) 5,0 g
- Séricité enrobée ("NOVATECH NFP Triple treated sericite" de DAIKIN) 2,0 g
- Poudre de nylon (ORGASOL ® 4000 d'ATOCHEM) 7,9 g

* poly méthyltrifluoropropyl diméthylsiloxane

Ce fond de teint appliqué sur le visage permet d'obtenir un maquillage homogène, absorbant bien le sébum au cours de la journée.

## Revendications

1. Composition cosmétique comprenant une première poudre absorbant le sébum ayant une prise de sébum, une deuxième poudre ayant une énergie de surface critique inférieure ou égale à 24 mN/m, et une phase grasse liquide liante comprenant une première huile non volatile ayant des paramètres de solubilité de Hansen $\delta^L_d$, $\delta^L_p$, $\delta^L_h$, exprimés en $J^{1/2}.\ cm^{-3/2}$, tels que

$$\Delta\delta = \sqrt{4\left(16.4 - \delta_d^L\right)^2 + \left(0.9 - \delta_p^L\right)^2 + \left(4.2 - \delta_h^L\right)^2}$$

et $\Delta\delta$ va de 6 à 20.

2. Composition selon la revendication 1, **caractérisée par le fait que** la première poudre a une prise de sébum supérieure ou égale à 1 ml/g, de préférence supérieure ou égale à 1,5 ml/g, et préférentiellement supérieure ou égale à 2 ml/g.

3. Composition selon la revendication 1 ou 2, **caractérisée par le fait que** la poudre absorbant le sébum a une surface spécifique supérieure ou égale à 300 $m^2/g$, de préférence supérieure à 500 $m^2/g$, et préférentiellement supérieure à 600 $m^2/g$.

4. Composition selon l'une quelconque des revendications précédentes, **caractérisée par le fait que** la poudre absorbant le sébum est choisi parmi la silice, les poudres de polymères acryliques, les poudres de polyamides, les poudres de silicone élastomère.

5. Composition selon l'une quelconque des revendications précédentes, **caractérisée par le fait que** la poudre absorbant le sébum est choisie parmi les poudres de polyméthacrylate de méthyle, de poly méthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle.

6. Composition selon l'une quelconque des revendications 1 à 4, **caractérisée par le fait que** la poudre absorbant le sébum est une poudre de silice.

7. Composition selon l'une quelconque des revendications précédentes, **caractérisée par le fait que** la poudre absorbant le sébum est présente en une teneur allant de 1 % à 98 % en poids, par rapport au poids total de la composition, de préférence allant de 1 % à 80 % en poids, et préférentiellement allant de 1 % à 60 % en poids, et plus préférentiellement allant de 1 % à 35 % en poids, et encore plus préférentiellement allant de 1 % à 15 % en poids.

8. Composition selon l'une quelconque des revendications précédentes, **caractérisée par le fait que** la deuxième poudre a une a une énergie de surface critique allant de 10 à 24 mN/m.

9. Composition selon l'une quelconque des revendications précédentes, **caractérisée par le fait que** la deuxième poudre a une énergie de surface critique inférieure ou égale à 20 mN/m, de préférence allant de 10 à 20 mN/m.

10. Composition selon l'une quelconque des revendications précédentes, **caractérisée par le fait que** la deuxième poudre est une poudre enrobée d'un composé organique fluoré ou d'un composé siliconé.

11. Composition selon la revendication précédente, **caractérisée par le fait que** le composé organique fluoré est un perfluoroalkylphosphate ou un polyoxyde d'hexafluoropropylène.

12. Composition selon la revendication précédente, **caractérisée par le fait que** le perfluoroalkyle phosphate est choisi parmi ceux de formule (I) :

$$[C_mF_{2m+1}C_nH_{2n}O]_yPO(OM)_{3-y}$$

avec m entier allant de 1 à 21 ; n entier allant de 1 à 14 ; y = 1, 2 ou 3 ; M est -H, un cation de métal alcalin, un groupe ammonium ou un groupe ammonium substitué par un à trois radicaux alkyle en $C_1$-$C_8$.

13. Composition selon la revendication 10, **caractérisée par le fait que** composé siliconé est choisi parmi les méthicones, les diméthicones, les polyorganosiloxanes comprenant des groupes perfluoroalkyles perfluoropolyéthers, les perfluoroalkyl silanes.

14. Composition selon l'une quelconque des revendications 10 à 13, **caractérisée par le fait que** le composé organique fluoré ou le composé siliconé enrobant la poudre est présent en une teneur allant de 0,01 % à 60 % en poids, par rapport au poids total de la poudre enrobée, de préférence allant de 0,05 % à 40 % en poids, et préférentiellement allant de 0,1 % à 40 % en poids.

15. Composition selon l'une quelconque des revendications précédentes, **caractérisée par le fait que** la deuxième poudre est présente en une teneur allant de 0,1 % à 95 % en poids, par rapport au poids total de la composition,

de préférence allant de 0,5 % à 90 % en poids, et préférentiellement allant de 5 % à 80 % en poids.

**16.** Composition selon l'une quelconque des revendications précédentes, **caractérisée par le fait que** la première huile non volatile a une viscosité, mesurée à 25 °C, supérieure ou égale à 5 x $10^{-2}$ Pa.s (50 cps), notamment allant de 5 x $10^{-2}$ Pa.s à 40 Pa.s (40 000 cps ), et de préférence supérieure ou égale à 9 x $10^{-2}$ Pa.s (90 cps), notamment allant de 9 x $10^{-2}$ Pa.s à 40 Pa.s.

**17.** Composition selon l'une quelconque des revendications précédentes, **caractérisée par le fait que** la première huile non volatile a des paramètres de solubilité de Hansen $\delta^L_d$, $\delta^L_p$, $\delta^L_h$ , exprimés en $J^{1/2}$. $cm^{-3/2}$ , tels que $\Delta\delta$ va de 6 à 16.

**18.** Composition selon l'une des revendications précédentes, **caractérisée par le fait que** la première huile non volatile est telle que $\delta^L_d$ est inférieur ou égal à 17, de préférence inférieur ou égal à 14.

**19.** Composition selon l'une quelconque des revendications précédentes, **caractérisée par le fait que** la première huile non volatile est choisie dans le groupe formé par le citrate de tri-iso arachidyle , le poly vinylpyrrolidone / hexadécène de poids moléculaire moyen en poids allant de 5000 à 9000, le trimellitate de butyl-2 octanol , le citrate de triisostéaryle, le trimellitate de tridécyle, le polyglycéryl-2 triisostéarate, le tétra-éthyl-2 hexanoate de pentaérythrityle, le trimellitate de triisodécyle, le malate de diisostéaryle, le trimellitate de tri-éthyl-2-hexyle, le 2-octyldodécanol, l'octyl hydroxystéarate, le polybutylène de poids moléculaire moyen en poids allant de 800 à 1200, le lactate d'isostéaryle, le monoisostéarate de propylène glycol, le polyglycéryl-2 diisostéarate, l'huile de ricin, le dibenzoate de dipropylène glycol, le triacétate de glycéryle oxyéthyléné (7 OE), le polyglycéryl-3 diisostéarate, le poly méthylfluoroalkyl diméthylsiloxane de formule (I) :

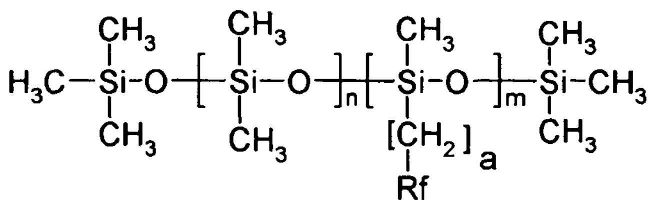

(I)

dans laquelle n est un entier allant de 5 à 90 et m est un entier allant de 1 à 150, a est un entier allant de 0 à 5, Rf désigne un radical perfluoroalkyle ayant de 1 à 8 atomes de carbone.

**20.** Composition selon l'une quelconque des revendications précédentes, **caractérisée par le fait que** l'huile non volatile est un poly méthylfluoroalkyl diméthylsiloxane de formule (I) :

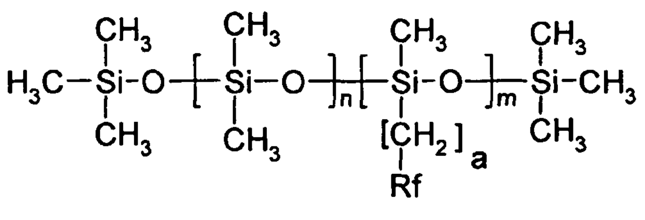

(I)

dans laquelle n est un entier allant de 5 à 90 et m est un entier allant de 1 à 150, a est un entier allant de 0 à 5, Rf désigne un radical perfluoroalkyle ayant de 1 à 8 atomes de carbone.

**21.** Composition selon l'une quelconque des revendications précédentes, **caractérisée par le fait que** la première

huile non volatile est présente en une teneur allant de 0,1 % à 90 % en poids, par rapport au poids total de la composition, de préférence allant de 0,1 % à 75 % en poids, et préférentiellement allant de 1 % à 50 % en poids, et mieux allant de 1 % à 30 % en poids.

**22.** Composition selon l'une quelconque des revendications précédentes, **caractérisée par le fait que** la phase grasse liquide comprend 100 % en poids de la première huile non volatile.

**23.** Composition selon l'une quelconque des revendications précédentes, **caractérisée par le fait que** la phase grasse liquide comprend une deuxième huile non volatile ou un mélange de deuxièmes huiles non volatiles.

**24.** Composition selon l'une quelconque des revendications précédentes, **caractérisée par le fait que** la phase grasse liquide comprend au moins une huile volatile.

**25.** Composition selon la revendication précédente, **caractérisée par le fait que** l'huile volatile est choisie parmi les huiles de silicones linéaires ou cycliques ayant notamment de 2 à 7 atomes de silicium, ces silicones comportant éventuellement des groupes alkyle ou alkoxy ayant de 1 à 10 atomes de carbone, les huiles volatiles hydrocarbonées ayant de 8 à 16 atomes de carbone.

**26.** Composition selon la revendication 24 ou 25, **caractérisée par le fait que** l'huile volatile est choisie parmi l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane, l'isododécane, l'isodécane, l'isohexadécane.

**27.** Composition selon l'une quelconque des revendications 24 à 26, **caractérisée par le fait que** l'huile volatile est présente en une teneur allant de 0,1 % à 90 % en poids, par rapport au poids total de la composition, de préférence allant de 0,1 % à 75 % en poids, et préférentiellement allant de 1 % à 50 % en poids.

**28.** Composition selon l'une quelconque des revendications précédentes, **caractérisée par le fait que** la phase liante comprend une phase aqueuse.

**29.** Composition selon la revendication précédente, **caractérisée par le fait que** la phase aqueuse est présente en une teneur allant de 0,1 % à 60 %, en poids, de préférence allant de 5 % à 50 % en poids, par rapport au poids total de la composition.

**30.** Composition selon l'une quelconque des revendications précédentes, **caractérisée par le fait qu'**elle comprend une poudre additionnelle différente des premières et deuxièmes poudres.

**31.** Composition selon la revendication précédente, **caractérisée par le fait que** la poudre additionnelle est choisie parmi les pigments, les nacres, les charges, et leurs mélanges.

**32.** Composition selon la revendication 30 ou 31, **caractérisée par le fait que** la poudre additionnelle est présente en une teneur allant de 0,01 % à 98,9 % en poids, par rapport au poids total de la composition, de préférence allant de 0,1 % à 85 % en poids, et préférentiellement allant de 1 % à 70 % en poids.

**33.** Composition selon l'une quelconque des revendications précédentes, **caractérisée par le fait qu'**elle comprend un ingrédient cosmétique choisi parmi les antioxydants, les parfums, les conservateurs, les neutralisants, les tensioactifs, les cires, les gélifiants, les polymères filmogènes, les épaississants, les filtres solaires, les vitamines, les hydratants, les composés auto-bronzants, les actifs antirides, et leurs mélanges.

**34.** Composition selon l'une quelconque des revendications précédentes, **caractérisée par le fait qu'**elle se présente sous forme de poudre compacte, de poudre coulée, de poudre libre, de gel, d'une émulsion eau-dans-huile, d'une émulsion huile-dans-eau, d'une émulsion multiple, d'un lait, d'une pâte.

**35.** Composition selon l'une quelconque des revendications 1 à 27 et 30 à 34, **caractérisée par le fait que** la composition est anhydre.

**36.** Composition selon l'une quelconque des revendications précédentes, **caractérisée par le fait que** se présente sous la forme d'un fond de teint, d'un fard à joue, d'un fard à paupières, d'un produit anti-cernes, d'une poudre du

visage et du corps, un produit de maquillage du corps.

**37.** Procédé cosmétique de maquillage ou de soin non thérapeutique des matières kératiniques comprenant l'application sur les matières kératiniques d'une composition selon l'une quelconque des revendications précédentes.

**38.** Utilisation d'une composition selon l'une quelconque des revendications 1 à 36 pour obtenir un maquillage de la peau non brillant et/ou homogène et/ou non transfert et/ou résistant aux frottements et/ou présentant un maintien de la couleur initiale au cours du temps.

**Claims**

**1.** Cosmetic composition comprising a first sebum-absorbing powder having a sebum uptake, a second powder having a critical surface energy of less than or equal to 24 mN/m, and a binding liquid fatty phase comprising a first non-volatile oil with Hansen solubility parameters $\delta^L{}_d$, $\delta^L{}_p$ and $\delta^L{}_h$, expressed in $J^{1/2}.cm^{-3/2}$, such that

$$\Delta\delta = \sqrt{4(16.4 - \delta_d^L)^2 + (0.9 - \delta_p^L)^2 + (4.2 - \delta_h^L)^2}$$

and $\Delta\delta$ ranges from 6 to 20.

**2.** Composition according to Claim 1, **characterized in that** the first powder has a sebum uptake of greater than or equal to 1 ml/g, preferably greater than or equal to 1.5 ml/g and preferentially greater than or equal to 2 ml/g.

**3.** Composition according to Claim 1 or 2, **characterized in that** the sebum-absorbing powder has a specific surface area of greater than or equal to 300 $m^2/g$, preferably greater than 500 $m^2/g$ and preferentially greater than 600 $m^2/g$.

**4.** Composition according to any one of the preceding claims, **characterized in that** the sebum-absorbing powder is chosen from silica, acrylic polymer powders, polyamide powders and silicone elastomer powders.

**5.** Composition according to any one of the preceding claims, **characterized in that** the sebum-absorbing powder is chosen from polymethyl methacrylate powder, polymethyl methacrylate/ethylene glycol dimethacrylate powder, polyallyl methacrylate/ethylene glycol dimethacrylate powder and ethylene glycol dimethacrylate/lauryl methacrylate copolymer powder.

**6.** Composition according to any one of Claims 1 to 4, **characterized in that** the sebum-absorbing powder is a silica powder.

**7.** Composition according to any one of the preceding claims, **characterized in that** the sebum-absorbing powder is present in a content ranging from 1% to 98% by weight, preferably ranging from 1% to 80% by weight, preferentially ranging from 1% to 60% by weight, more preferentially ranging from 1% to 35% by weight and even more preferentially ranging from 1% to 15% by weight, relative to the total weight of the composition.

**8.** Composition according to any one of the preceding claims, **characterized in that** the second powder has a critical surface energy ranging from 10 to 24 mN/m.

**9.** Composition according to any one of the preceding claims, **characterized in that** the second powder has a critical surface energy of less than or equal to 20 mN/m and preferably ranging from 10 to 20 mN/m.

**10.** Composition according to any one of the preceding claims, **characterized in that** the second powder is a powder coated with an organofluorine compound or a silicone compound.

**11.** Composition according to the preceding claim, **characterized in that** the organofluorine compound is a perfluoroalkyl phosphate or a polyhexafluoropropylene oxide.

**12.** Composition according to the preceding claim, **characterized in that** the perfluoroalkyl phosphate is chosen from

those of formula (I):

$$[C_mF_{2m+1}C_nH_{2n}O]_yPO(OM)_{3-y}$$

with m being an integer ranging from 1 to 21; n an integer from 1 to 14; y = 1, 2 or 3; M is -H, an alkali metal cation, an ammonium group or an ammonium group substituted with 1 to 3 $C_1$-$C_8$ alkyl radicals.

**13.** Composition according to Claim 10, **characterized in that** the silicone compound is chosen from methicones, dimethicones, polyorganosiloxanes comprising perfluoroalkyl or perfluoropolyether groups, and perfluoroalkylsilanes.

**14.** Composition according to any one of Claims 10 to 13, **characterized in that** the organofluorine compound or the silicone compound coating the powder is present in a content ranging from 0.01% to 60% by weight, preferably ranging from 0.05% to 40% by weight and preferentially ranging from 0.1% to 40% by weight, relative to the total weight of the coated powder.

**15.** Composition according to any one of the preceding claims, **characterized in that** the second powder is present in a content ranging from 0.1% to 95% by weight, preferably ranging from 0.5% to 90% by weight and preferentially ranging from 5% to 80% by weight, relative to the total weight of the composition.

**16.** Composition according to any one of the preceding claims, **characterized in that** the first non-volatile oil has a viscosity, measured at 25°C, of greater than or equal to $5 \times 10^{-2}$ Pa.s (50 cps), especially ranging from $5 \times 10^{-2}$ Pa.s to 40 Pa.s (40 000 cps) and preferably greater than or equal to $9 \times 10^{-2}$ Pa.s (90 cps), especially ranging from $9 \times 10^{-2}$ Pa.s to 40 Pa.s.

**17.** Composition according to any one of the preceding claims, **characterized in that** the first non-volatile oil has Hansen solubility parameters $\delta^L_d$, $\delta^L_p$ and $\delta^L_h$, expressed in $J^{1/2}.cm^{-3/2}$, such that $\Delta\delta$ ranges from 6 to 16.

**18.** Composition according to one of the preceding claims, **characterized in that** the first non-volatile oil is such that $\delta^L_d$ is less than or equal to 17 and preferably less than or equal to 14.

**19.** Composition according to any one of the preceding claims, **characterized in that** the first non-volatile oil is chosen from the group formed by triisoarachidyl citrate, polyvinylpyrrolidone/hexadecene with a weight-average molecular weight ranging from 5000 to 9000, 2-butyloctyl trimellitate, triisostearyl citrate, tridecyl trimellitate, polyglyceryl-2 triisostearate, pentaerythrityl tetrakis(2-ethylhexanoate), triisodecyl trimellitate, diisostearyl malate, tris(2-ethylhexyl) trimellitate, 2-octyldodecanol, octyl hydroxystearate, polybutylene with a weight-average molecular weight ranging from 800 to 1200, isostearyl lactate, propylene glycol monoisostearate, polyglyceryl-2 diisostearate, castor oil, dipropylene glycol dibenzoate, oxyethylenated (7 EO) glyceryl triacetate, polyglyceryl-3 diisostearate and the polymethylfluoroalkyldimethylsiloxane of formula (I) :

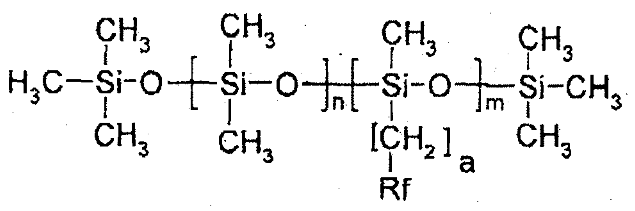

(I)

in which n is an integer ranging from 5 to 90 and m is an integer ranging from 1 to 150, a is an integer ranging from 0 to 5 and Rf denotes a perfluoroalkyl radical containing from 1 to 8 carbon atoms.

**20.** Composition according to any one of the preceding claims, **characterized in that** the non-volatile oil is a polymethylfluoroalkyldimethylsiloxane of formula (I):

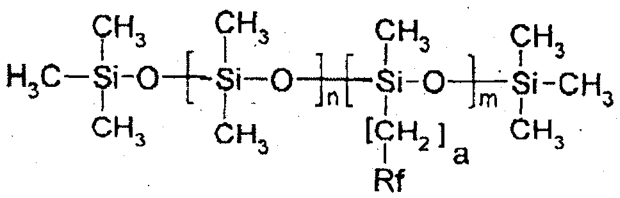

(I)

in which n is an integer ranging from 5 to 90 and m is an integer ranging from 1 to 150, a is an integer ranging from 0 to 5 and Rf denotes a perfluoroalkyl radical containing from 1 to 8 carbon atoms.

**21.** Composition according to any one of the preceding claims, **characterized in that** the first non-volatile oil is present in a content ranging from 0.1% to 90% by weight, preferably ranging from 0.1% to 75% by weight, preferentially ranging from 1% to 50% by weight and better still ranging from 1% to 30% by weight, relative to the total weight of the composition.

**22.** Composition according to any one of the preceding claims, **characterized in that** the liquid fatty phase comprises 100% by weight of the first non-volatile oil.

**23.** Composition according to any one of the preceding claims, **characterized in that** the liquid fatty phase comprises a second non-volatile oil or a mixture of second non-volatile oils.

**24.** Composition according to any one of the preceding claims, **characterized in that** the liquid fatty phase comprises at least one volatile oil.

**25.** Composition according to the preceding claim, **characterized in that** the volatile oil is chosen from linear or cyclic silicone oils especially containing from 2 to 7 silicon atoms, these silicones optionally comprising alkyl or alkoxy groups containing from 1 to 10 carbon atoms, and hydrocarbon-based volatile oils containing from 8 to 16 carbon atoms.

**26.** Composition according to Claim 24 or 25, **characterized in that** the volatile oil is chosen from octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane, dodecamethylpentasiloxane isododecane, isodecane and isohexadecane.

**27.** Composition according to any one of Claims 24 to 26, **characterized in that** the volatile oil is present in a content ranging from 0.1% to 90% by weight, preferably ranging from 0.1% to 75% by weight and preferentially ranging from 1% to 50% by weight, relative to the total weight of the composition.

**28.** Composition according to any one of the preceding claims, **characterized in that** the binder phase comprises an aqueous phase.

**29.** Composition according to the preceding claim, **characterized in that** the aqueous phase is present in a content ranging from 0.1% to 60% by weight and preferably ranging from 5% to 50% by weight, relative to the total weight of the composition.

**30.** Composition according to any one of the preceding claims, **characterized in that** it comprises an additional powder that is different from the first and second powders.

**31.** Composition according to the preceding claim, **characterized in that** the additional powder is chosen from pigments, nacres and fillers, and mixtures thereof.

**32.** Composition according to Claim 30 or 31, **characterized in that** the additional powder is present in a content ranging from 0.01% to 98.9% by weight, preferably ranging from 0.1% to 85% by weight and preferentially ranging from 1%

to 70% by weight, relative to the total weight of the composition.

33. Composition according to any one of the preceding claims, **characterized in that** it comprises a cosmetic ingredient chosen from antioxidants, fragrances, preserving agents, neutralizers, surfactants, waxes, gelling agents, film-forming polymers, thickeners, sunscreens, vitamins, moisturizers, self-tanning compounds and antiwrinkle active agents, and mixtures thereof.

34. Composition according to any one of the preceding claims, **characterized in that** it is in the form of a compact powder, a cast powder, a free powder, a gel, a water-in-oil emulsion, an oil-in-water emulsion, a multiple emulsion, a milk or a paste.

35. Composition according to any one of Claims 1 to 27 and 30 to 34, **characterized in that** the composition is anhydrous.

36. Composition according to any one of the preceding claims, **characterized in that** it is in the form of a foundation, a rouge, an eyeshadow, a concealer product, a face and body powder or a body makeup product.

37. Non-therapeutic cosmetic process for making up or caring for keratin materials, comprising the application to the keratin materials of a composition according to any one of the preceding claims.

38. Use of a composition according to any one of Claims 1 to 36, to obtain a non-shiny and/or uniform and/or transfer-resistant and/or rub-resistant makeup result on the skin and/or a makeup result that shows maintenance of the initial colour over time.

## Patentansprüche

1. Kosmetische Zusammensetzung, die ein erstes Sebumabsorbierendes Pulver, das Sebum aufnimmt, ein zweites Pulver, das eine kritische Oberflächenenergie von kleiner oder gleich 24 mN/m aufweist, und eine bindende flüssige Fettphase enthält, die ein erstes nicht flüchtiges Öl enthält, das in $J^{1/2} \cdot cm^{-3/2}$ ausgedrückte Solubilitätsparameter nach Hansen $\delta^L_d$, $\delta^L_p$, $\delta^L_h$ aufweist, die so sind, dass:

$$\Delta\delta = \sqrt{4\,(16.4 - \delta^L_d)^2 + (0.9 - \delta^L_p)^2 + (4.2 - \delta^L_h)^2}$$

und $\Delta\delta$ im Bereich von 6 bis 20 liegt.

2. Zusammensetzung nach Anspruch 1, **dadurch gekennzeichnet, dass** das erste Pulver eine Sebumaufnahme von größer oder gleich 1 ml/g, vorzugsweise von größer oder gleich 1,5 ml/g und bevorzugt von größer oder gleich 2 ml/g aufweist.

3. Zusammensetzung nach Anspruch 1 oder 2, **dadurch gekennzeichnet, dass** das Sebum-absorbierende Pulver eine spezifische Oberfläche von mindestens 300 $m^2/g$, vorzugsweise über 500 $m^2/g$ und bevorzugt über 600 $m^2/g$ über aufweist.

4. Zusammensetzung nach einem der vorhergehenden Ansprüche, **dadurch gekennzeichnet, dass** das Sebum-absorbierende Pulver unter Kieselsäure, Acrylpolymerpulvern, Polyamidpulvern und Pulvern aus einem Siliconelastomer ausgewählt sind.

5. Zusammensetzung nach einem der vorhergehenden Ansprüche, **dadurch gekennzeichnet, dass** das Sebum-absorbierende Pulver unter den Pulvern von Polymethylmethacrylat, Pulvern von Polymethylmethacrylat/Ethylenglycoldimethacrylat, Pulvern von Polyallylmethacrylat/Ethylenglycoldimethacrylat, Pulvern von Ethylenglycoldimethacrylat/Laurylmethacrylat-Copolymer ausgewählt ist.

6. Zusammensetzung nach einem der Ansprüche 1 bis 4, **dadurch gekennzeichnet, dass** das Sebum-absorbierende Pulver ein Kieselsäurepulver ist.

7. Zusammensetzung nach einem der vorhergehenden Ansprüche, **dadurch gekennzeichnet, dass** das Sebum-absorbierende Pulver in einem Mengenanteil im Bereich von 1 bis 98 Gew.-%, bezogen auf das Gesamtgewicht der Zusammensetzung, vorzugsweise im Bereich von 1 bis 80 Gew.-%, bevorzugt im Bereich von 1 bis 60 Gew.-%, besonders bevorzugt im Bereich von 1 bis 35 Gew.-% und noch bevorzugter im Bereich von 1 bis 15 Gew.-% vorliegt.

8. Zusammensetzung nach einem der vorhergehenden Ansprüche, **dadurch gekennzeichnet, dass** das zweite Pulver eine kritische Oberflächenenergie im Bereich von 10 bis 24 mN/m aufweist.

9. Zusammensetzung nach einem der vorhergehenden Ansprüche, **dadurch gekennzeichnet, dass** das zweite Pulver eine kritische Oberflächenenergie von höchstens 20 mN/m und vorzugsweise im Bereich von 10 bis 20 mN/m aufweist.

10. Zusammensetzung nach einem der vorhergehenden Ansprüche, **dadurch gekennzeichnet, dass** das zweite Pulver ein Pulver ist, das mit einer fluorierten organischen Verbindung oder einer Siliconverbindung umhüllt ist.

11. Zusammensetzung nach dem vorhergehenden Anspruch, **dadurch gekennzeichnet, dass** die fluorierte organische Verbindung ein Perfluoralkylphosphat oder ein Hexafluorpropylenpolyoxid ist.

12. Zusammensetzung nach dem vorhergehenden Anspruch, **dadurch gekennzeichnet, dass** das Perfluoralkylphosphat unter den Verbindungen der Formel (I) ausgewählt ist:

$$[C_mF_{2m+1}C_nH_{2n}O]_yPO(OM)_{3-y}$$

wobei m im Bereich von 1 bis 21 liegt; n eine ganze Zahl im Bereich von 1 bis 14 ist; y = 1, 2 oder 3; und M -H, ein Alkalimetallkation, eine Ammoniumgruppe oder eine mit 1 bis 3 $C_{1-8}$-Alkylgruppen substituierte Ammoniumgruppe bedeutet.

13. Zusammensetzung nach Anspruch 10, **dadurch gekennzeichnet, dass** die Siliconverbindung unter den Methiconen, Dimethiconen, Polyorganosiloxanen, die Perfluoralkylperfluorpolyethergruppen aufweisen, und Perfluoralkylsilanen ausgewählt ist.

14. Zusammensetzung nach einem der Ansprüche 10 bis 13, **dadurch gekennzeichnet, dass** die fluorierte organische Verbindung oder die Siliconverbindung, die das Pulver umhüllen, in einer Menge von 0,01 bis 60 Gew.-%, bezogen auf das Gesamtgewicht des umhüllten Pulvers, vorzugsweise im Bereich von 0,05 bis 40 Gew.-% und bevorzugt im Bereich von 0,1 bis 40 Gew.-% enthalten ist.

15. Zusammensetzung nach einem der vorhergehenden Ansprüche, **dadurch gekennzeichnet, dass** das zweite Pulver in einem Mengenanteil von 0,1 bis 95 Gew.-%, bezogen auf das Gesamtgewicht der Zusammensetzung, vorzugsweise im Bereich von 0,5 bis 90 Gew.-% und bevorzugt im Bereich von 5 bis 80 Gew.-% vorliegt.

16. Zusammensetzung nach einem der vorhergehenden Ansprüche, **dadurch gekennzeichnet, dass** das erste, nicht flüchtige Öl eine bei 25 °C gemessene Viskosität von 5 x $10^{-2}$ Pa·s (50 cP) oder darüber, insbesondere im Bereich von 5 x $10^{-2}$ bis 40 Pa.s (40000 cP), vorzugsweise 9 x $10^{-2}$ Pa.s (90 cP) oder darüber und besonders im Bereich von 9 x $10^{-2}$ bis 40 Pa.s aufweist.

17. Zusammensetzung nach einem der vorhergehenden Ansprüche, **dadurch gekennzeichnet, dass** das erste, nicht flüchtige Öl in $J^{1/2}\cdot cm^{-3/2}$ ausgedrückte Solubilitätsparameter nach Hansen $\delta^L_d$, $\delta^L_p$, $\delta^L_h$ aufweist, die so sind, dass $\Delta\delta$ im Bereich von 6 bis 16 liegt.

18. Zusammensetzung nach einem der vorhergehenden Ansprüche, **dadurch gekennzeichnet, dass** das erste, nicht flüchtige Öl so ist, dass $\delta^L_d$ kleiner oder gleich 17 und vorzugsweise kleiner oder gleich 14 ist.

19. Zusammensetzung nach einem der vorhergehenden Ansprüche, **dadurch gekennzeichnet, dass** das erste, nicht flüchtige Öl unter Triisoarachidylcitrat, Polyvinylpyrrolidon/Hexadecen mit einer gewichtsmittleren Molmasse im Bereich von 5000 bis 9000, 2-Butyloctanol-trimellitat, Triisostearylcitrat, Tridecyltrimellitat, Polyglyceryl-2 Triisostearate, Pentaerythrityl-tetra-2-ethyl-hexanoat, Triisodecyltrimellitat, Diisostearylmalat, Tri-2-ethylhexyltrimellitat, 2-Octyldodecanol, Octylhydroxystearat, Polybutylen mit einer gewichtsmittleren Molmasse von 800 bis 1200, Isostearyllactat, Propylenglycolmonoisostearat, Polyglyceryl-2 Diisostearate, Ricinusöl, Dipropylenglycoldibenzoat, ethoxyliertem (7 EO) Glyceryltriacetat, Polyglyceryl-3 Diisostearate, Polymethylfluoralkyldimethylsiloxan der Formel (I) ausgewählt ist:

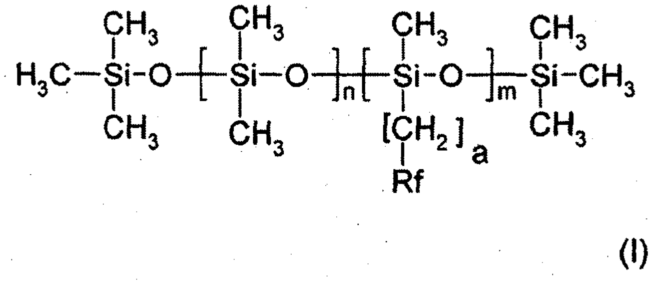

(I)

worin n eine ganze Zahl von 5 bis 90 bedeutet, m eine ganze Zahl von 1 bis 150 ist, a eine ganze Zahl von 0 bis 5 bedeutet und Rf eine Perfluoralkylgruppe mit 1 bis 8 Kohlenstoffatomen ist.

**20.** Zusammensetzung nach einem der vorhergehenden Ansprüche, **dadurch gekennzeichnet, dass** das nicht flüchtige Öl ein Polymethylfluoralkyldimethylsiloxan der Formel (I) ist:

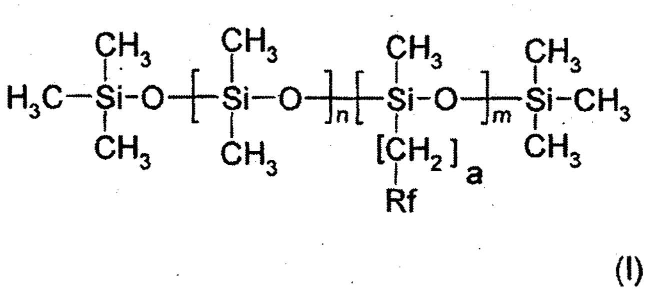

(I)

worin n eine ganze Zahl von 5 bis 90 bedeutet, m eine ganze Zahl von 1 bis 150 ist, a eine ganze Zahl von 0 bis 5 bedeutet und Rf eine Perfluoralkylgruppe mit 1 bis 8 Kohlenstoffatomen ist.

**21.** Zusammensetzung nach einem der vorhergehenden Ansprüche, **dadurch gekennzeichnet, dass** das erste, nicht flüchtige Öl in einem Mengenanteil im Bereich von 0,1 bis 90 Gew.-%, bezogen auf das Gesamtgewicht der Zusammensetzung, vorzugsweise im Bereich von 0,1 bis 75 Gew.-%, bevorzugt im Bereich von 1 bis 50 Gew.-% und besser im Bereich von 1 bis 30 Gew.-% enthalten ist.

**22.** Zusammensetzung nach einem der vorhergehenden Ansprüche, **dadurch gekennzeichnet, dass** die flüssige Fettphase 100 Gew.-% des ersten, nicht flüchtigen Öls enthält.

**23.** Zusammensetzung nach einem der vorhergehenden Ansprüche, **dadurch gekennzeichnet, dass** die flüssige Fettphase ein zweites, nicht flüchtiges Öl oder ein Gemisch von zweiten, nicht flüchtigen Ölen enthält.

**24.** Zusammensetzung nach einem der vorhergehenden Ansprüche, **dadurch gekennzeichnet, dass** die flüssige Fettphase mindestens ein flüchtiges Öl enthält.

**25.** Zusammensetzung nach dem vorhergehenden Anspruch, **dadurch gekennzeichnet, dass** das flüchtige Öl unter den linearen oder cyclischen Siliconen mit 2 bis 7 Siliciumatomen, wobei diese Silicone gegebenenfalls Alkyl- oder Alkoxygruppen mit 1 bis 10 Kohlenstoffatomen aufweisen, und flüchtigen Kohlenwasserstoffölen mit 8 bis 16 Kohlenstoffatomen ausgewählt ist.

**26.** Zusammensetzung nach Anspruch 24 oder 25, **dadurch gekennzeichnet, dass** das flüchtige Öl unter Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan, Isododecan, Isodecan und Isohexadecan ausgewählt ist.

**27.** Zusammensetzung nach einem der Ansprüche 24 bis 26, **dadurch gekennzeichnet, dass** das flüchtige Öl in einem Mengenanteil im Bereich von 0,1 bis 90 Gew.-%, bezogen auf das Gesamtgewicht der Zusammensetzung, vorzugsweise im Bereich von 0,1 bis 75 Gew.-% und bevorzugt im Bereich von 1 bis 50 Gew.-% enthalten ist.

**28.** Zusammensetzung nach einem der vorhergehenden Ansprüche, **dadurch gekennzeichnet, dass** die bindende Phase eine wässrige Phase umfasst.

**29.** Zusammensetzung nach dem vorhergehenden Anspruch, **dadurch gekennzeichnet, dass** die wässrige Phase in einem Mengenanteil im Bereich von 0,1 bis 60 Gew.-% und vorzugsweise 5 bis 50 Gew.-%, bezogen auf das Gesamtgewicht der Zusammensetzung, vorliegt.

**30.** Zusammensetzung nach einem der vorhergehenden Ansprüche, **dadurch gekennzeichnet, dass** sie ferner eine ergänzendes, von dem ersten und zweiten Pulver verschiedenes Pulver enthält.

**31.** Zusammensetzung nach dem vorhergehenden Anspruch, **dadurch gekennzeichnet, dass** das ergänzende Pulver unter den Pigmenten, Perlglanzstoffen, Füllstoffen und deren Gemischen ausgewählt ist.

**32.** Zusammensetzung nach Anspruch 30 oder 31, **dadurch gekennzeichnet, dass** das ergänzende Pulver in einer Menge im Bereich von 0,01 bis 98,9 Gew.-%, bezogen auf das Gesamtgewicht der Zusammensetzung, vorzugsweise im Bereich von 0,1 bis 85 Gew.-% und bevorzugt im Bereich von 1 bis 70 Gew.-% vorliegt.

**33.** Zusammensetzung nach einem der vorhergehenden Ansprüche, **dadurch gekennzeichnet, dass** sie einen kosmetischen Bestandteil enthält, der unter den Antioxidantien, Parfums, Konservierungsmitteln, Neutralisationsmitteln, grenzflächenaktiven Stoffen, Wachsen, Gelbildnern, filmbildenden Polymeren, Verdickungsmitteln, Sonnenschutzfiltern, Vitaminen, Hydratisierungsmitteln, Selbstbräunungsmitteln, Wirkstoffen gegen Falten und deren Gemischen ausgewählt ist.

**34.** Zusammensetzung nach einem der vorhergehenden Ansprüche, **dadurch gekennzeichnet, dass** sie als kompakte Pulver, gegossenes Pulver, loses Pulver, Gel, Wasser-in-Öl-Emulsion, Ölin-Wasser-Emulsion, multiple Emulsion, Milch oder Paste vorliegt.

**35.** Zusammensetzung nach einem der Ansprüche 1 bis 27 und 30 bis 34, **dadurch gekennzeichnet, dass** die Zusammensetzung wasserfrei ist.

**36.** Zusammensetzung nach einem der vorhergehenden Ansprüche, **dadurch gekennzeichnet, dass** sie als Make-up, Wangenrouge, Lidschatten, Produkt gegen Augenringe, Gesichts- und Körperpuder, Produkt zum Schminken des Körpers vorliegt.

**37.** Kosmetisches Verfahren zum Schminken oder die nicht therapeutische Pflege von Keratinsubstanzen, das das Auftragen einer Zusammensetzung nach einem der vorhergehenden Ansprüche auf die Keratinsubstanzen umfasst.

**38.** Verwendung einer Zusammensetzung nach einem der Ansprüche 1 bis 36, um auf der Haut eine nicht glänzende Schminke und/oder homogene Schminke und/oder sich nicht übertragende Schminke und/oder reibungsbeständige Schminke und/oder Schminke zu bilden, die die anfängliche Farbe im Laufe der Zeit nicht verliert.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9704737 A
- WO 9617583 A
- JP 200055134 B
- EP 1116753 A
- US 3632744 A
- JP 4330007 A
- JP 2001316223 A


### Littérature non-brevet citée dans la description


- *The journal of the American Chemical Society,* Février 1938, vol. 60, 309